# EUROPEAN PATENT SPECIFICATION

(11) **EP 2 056 921 B1**
(45) Date of publication and mention of the grant of the patent: **18.12.2019**
(21) Application number: 06851869.5
(22) Date of filing: 29.08.2006
(51) Int. Cl.: A61M 37/00, A61M 35/00, A61N 1/30, A61N 1/40, A61B 17/20, A61B 10/00

(54) **HIGH-ASPECT-RATIO MICRODEVICES FOR TRANSDERMAL DELIVERY AND SAMPLING OF ACTIVE SUBSTANCES**
MIKROVORRICHTUNGEN MIT HOHEM ASPEKTVERHÄLTNIS FÜR DIE TRANSDERMALE ABGABE UND PROBENENTNAHME VON WIRKSTOFFEN
MICRO-DISPOSITIFS À RAPPORT DIMENSIONNEL ÉLEVÉ POUR ADMINISTRATION TRANSDERMIQUE ET ÉCHANTILLONNAGE DE SUBSTANCES ACTIVES

(43) Date of publication of application: 13.05.2009
(73) Proprietor: Nanomed Devices, Inc., Slingerlands, NY 12159 (US)
(72) Inventor: XU, Bai, Slingerlands, NY 12159 (US)
(74) Representative: Westphal, Mussgnug & Partner Patentanwälte mbB
(86) International application number: PCT/US2006/033877
(87) International publication number: WO 2008/054362

(56) References cited:
- US-A1- 2002 095 134
- US-A1- 2002 099 356
- US-A1- 2005 000 514
- US-A1- 2005 261 632
- US-B1- 6 503 231

## Description

### FIELD OF THE INVENTION

The present invention relates to high-aspect-ratio microdevices and the method of making and using the same. The microdevices referred here include microneedles, microneedle arrays, microblades, microblade arrays, microknives, and microknife arrays.

### BACKGROUND OF THE INVENTION

Drugs are commonly administered in solid form through pills or capsules that can be orally taken. However, many biological drugs can not be administered this way because of degradation in the gastrointestinal tract and quick elimination by the liver. Another common technique for administration of drugs in liquid form is through injection using a metal hypodermic needle that can cause significant pain and discomfort to patients. A number of physical and chemical techniques including electroporation, laser ablation, ultrasound, thermal, iontophoresis and chemical enhancers have been explored to develop painless transdermal drug delivery techniques. It was found that it is very difficult for the molecules with a molecular weight higher than 500 or diameter larger than 1 nm to penetrate normal human skin. Further studies showed that the key barrier for transdermal delivery of substances is the stratum corneum layer, the outer layer of skin, that is about 4-30 micron thick. Invasive methods to overcome this skin barrier have been used in practice, such as intradermal (ID), intramuscular (IM) or subcutaneous (SC) injection using standard hyperdomic needles and syringes. These methods cause pain and require a skilled professional. In addition, they may cause needle injuries. Similarly, current method of extracting biologic fluids such as blood from patients suffers from the same disadvantages.

In order to improve the skin permeability of the therapeutic agents and other active ingredients, microneedles have been recently developed to disrupt the stratum corneum and facilitate the delivery of the active agents and ingredients to the epidermis. These active substances can then diffuse through the rest of epidermis to the dermis and absorbed by blood vessels and lymphatics there. The substance absorbed can get into circulation system. Thus both topical and system-level delivery of drugs is possible. Since there are no nerves and blood vessels in stratum corneum and epidermis, this is a minimally invasive, painless and blood-free method of drug delivery. An additional advantage of this method, when engineered for topical delivery of vaccines, can lead to enhanced inoculation effect because the epidermis is rich in antigen presenting cells and is a desired target for vaccine delivery.

The prior art reports many devices and methods to overcome the skin barriers. For example, U.S. Patent No. 5,855,801 and U.S. Patent No. 5,928,207 assigned to The Regents of the University of California taught a microneedle fabrication method similar to IC compatible neural recording arrays. The disclosed microneedle arrays are typically linear array as they are in the plane of the silicon substrate surface. Microneedles have been also fabricated by heating the glass tube and lengthening the heated part till the diameter of the tip is reduced to the desired range. It is in general very difficult to control the size of the needle shaft and the tip this way although biologists are still using this method to produce microneedles that can inject or withdraw substances from a single cell.

U.S. Patent No. 6,503,231 by Prausnitz et al discloses a method for making out-of-the-plane porous or hollow microneedles. It either involves porous silicon formed by anodization of silicon or deals with sacrificial molds or selective removal of substrate materials to form fluidic conduits. U.S. Patent No. 6,511,463 by JDS Uniphase Corp. also taught a method to fabricate the same. U.S. Patent No. 6,558,361 assigned to Nanopass Ltd. taught a method for the manufacture of hollow microneedle arrays by removing a selective area of substrate material. U.S. Patent No. 6,603,987 assigned to Bayer Corp. also disclosed a method to make hollow microneedle patch. All these methods are trying to perform certain functions of the current hyperdomic needles and create a miniaturized analog to perform drug delivery or extract body fluids without causing pain and discomfort.

More recently, U.S. application publication No. 2004/0241965 discloses a method of making high aspect ratio electrode arrays comprised of solid metals. It involves the preparation of porous microchannel glass template, electrodeposition of metals in the microchannels, and final preparation of electrode array following electrodeposition. The body of microelectrode is formed by electrodeposition method similar to those used in forming nanowires. Some other arrays of microneedles are described in U.S. Patent Nos. 6743211; 6454755; 6611707; and 6471903. US 2002/099356 discloses a system for delivering an agent or a combination of agents, comprising a microdevice comprising an array of microstructures, wherein the microdevice has an aspect ratio of about 10:1 or higher,an application mechanism for applying the microdevice to an area of skin, wherein the application mechanism comprises a mechanical applicator, an ultrasound applicator, a mechanical vibrator, an iontophoresis device, a sonophoresis device, a radiofrequency (RF) device, or a heat generating device.

The prior methods to make microneedles, whether they are in-the-plane or out-of-the-plane from the substrate material, are cumbersome and expensive. The hollow microneedle arrays, while their sizes are scaled down from conventional needles, are especially expensive to make because of complexity in fabrication process. Their mechanical integrity also suffers as their sizes become smaller. Their tips are often no in nanoscale range, making it difficult to perforate stratum corneum.

Accordingly, a continuing need exists for an improved low cost, disposable transdermal delivery device for effective delivery of substances in a controlled manner.

### SUMMARY OF THE INVENTION

The present invention provides high-aspect-ratio microstructures (HARMS) and methods of using the same. The present invention also provides methods of using the device for transdermal delivery of drugs, vaccines, diagnostic agents and cosmetic substances and sampling of body fluids for treating , preventing, or ameliorating a medical condition of a mammal such as a human being. In some embodiments, the method comprises treating a topical site of a mammal using a device, and applying an effective amount of an agent to the topical site to allow the agent to penetrate into the body of the mammal. The device can comprise an array of microstructures. The microstructure can have an aspect ratio of about 5:1, 10:1, 15:1, 20:1 or higher.

Reference(s) to "embodiment(s)" throughout the description which are not under the scope of the appended claims merely represent possible exemplary executions and are therefore not part of the present invention.

In some embodiments, the present invention provides a system for topical delivery of an agent for a medical condition in a mammal (e.g., a patient). The system comprises: (1) a microdevice comprising an array of microstructures, (2) an application mechanism for applying the microdevice to an area of skin of a patient to generate a prepared area of skin comprising a plurality of nanopores or nanochannels in stratum corneum of the prepared area of skin, and (3) a delivery mechanism for causing the agent to be delivered to the mammal through the nanopores or nanochannels in the stratum corneum of the prepared area of skin. The microdevice can comprise nanoscale tips and microscale body that can have an aspect ratio of about 5:1, 10:1, 15:1, 20:1 or higher.

In some embodiments, the present invention provides a method of delivering an agent for a medical condition to a mammal. The method comprises: (1) applying an application mechanism to a microdevice to cause the microdevice to contact an area of skin to generate a prepared area of skin comprising a plurality of nanopores or nanochannels through the stratum corneum of the area of skin, (2) applying a composition comprising the agent to the prepared area of skin, and (3) causing an effective amount of the agent to deliver to the patient through the nanopores or nanochannels in the stratum corneum.

In some embodiments, the method of delivering an agent described herein includes: (1) applying a composition comprising the agent to an area of skin, (2) applying an application mechanism to a microdevice to cause the microdevice to contact the area of skin to generate a plurality of nanopores or nanochannels through the stratum corneum of the area of skin, and (3) causing an effective amount of the agent to deliver to the patient through the nanopores or nanochannels in the stratum corneum.

In some embodiments, the method of delivering an agent described herein includes: (1) applying an application mechanism to a microdevice to cause the microdevice to contact an area of skin to generate a prepared area of skin comprising a plurality of nanopores or nanochannels through the stratum corneum of the area of skin, and (2) causing an effective amount of an agent to deliver to the patient through the nanopores or nanochannels in the stratum corneum. The microstructure is coated with a composition comprising the agent.

### BRIEF DESCRIPTION OF THE DRAWINGS

Figure 1 is a schematic view of an embodiment of a microneedle array formed using the disclosed process flow illustrated in Figures 6A-6H.
Figure 2 is a scanning electron microscope micrograph of a microneedle array fabricated using the method disclosed in the Example 1 and illustrated in Figures 6A-6H.
Figure 3 is a scanning electron microscope micrograph of microneedle array fabricated using the method disclosed in the Example 2
Figures 4A and 4B are perspective illustration and side cross-sectional view of microknives.
Figures 5A and 5B are perspective illustration and side cross-sectional view of microblades.
Figures 6A to 6H are cross-sectional illustration of a fabrication process flow of a preferred method to form microdevices.
Figure 7 shows level of cumulative amount permeated through skin for pureriran with and without microneedle treatment in an *in vitro* test using the method disclosed in the current invention.
Figure 8 shows plasma concentration of fluorescence-labeled bovine serum albumin in rabbit tested *in vivo* at BSA loading in carbopol 934P of 0.15%, 0.20%, 0.25%, 0.30% (Weight/Volume) for each microneedle array that has 400 microneedles. The total composition amount is 1 mg on microneedle arrays.
Figure 9 shows level of cumulative amount permeated through skin measured *in vitro* for Botulinum Toxin Type A and Bovine Serum Albumin with microneedle treatment using the method disclosed in the current invention.
Figure 10 shows interferon-alfa-lb activity measured *in vivo* on rabbit with 4.8 x 10⁷ IU interferon-alpha-lb administered using method disclosed in the current invention as compared to subcutaneous injection of the same amount of interferon-alfa-lb.
Figure 11 shows that lidocaine, an anesthetic agent, can penetrate skin 10 times faster on a microneedle pre-treated skin.
Figure 12 shows the test result on 30 human subjects , demonstrating shows 7 times better in the improvement of the pain score with microneedle assisted lidocaine patch, compared to placebo group that has lidocaine patch only.
Figure 13 shows blood concentration of BSA indicated by fluorescence intensity, which was delivered to rabbit skin using a microneedle array of the present invention.

### DETAILED DESCRIPTION OF THE INVENTION

The present invention provides high-aspect-ratio microstructures (HARMS) and methods of using the same. The present invention also provides methods of using the device for transdermal delivery of drugs, vaccines, diagnostic agents and cosmetic substances and sampling of body fluids for treating , preventing, or ameliorating a medical condition of a mammal such as a human being. In some embodiments, the method comprises treating a topical site of a mammal using a device, and applying an effective amount of an agent to the topical site to allow the agent to penetrate into the body of the mammal. The device can include an array of microstructures. The microstructure can have an aspect ratio of about 5:1, 10:1, 15:1, 20:1 or higher.

In some embodiments, the present invention provides a system for topical delivery of an agent for a medical condition in a mammal (e.g., a patient). The system comprises: (1) a microdevice comprising an array of microstructures, (2) an application mechanism for applying the microdevice to an area of skin of a patient to generate a prepared area of skin comprising a plurality of nanopores or nanochannels in stratum corneum of the prepared area of skin, and (3) a delivery mechanism for causing the agent to be delivered to the mammal through the nanopores or nanochannels in the stratum corneum of the prepared area of skin. The microdevice can comprise nanoscale tips and microscale body that can have an aspect ratio of about 5:1, 10:1, 15:1, 20:1 or higher.

In some embodiments, the present invention provides a method of delivering an agent for a medical condition to a mammal. The method comprises: (1) applying an application mechanism to a microdevice to cause the microdevice to contact an area of skin to generate a prepared area of skin comprising a plurality of nanopores or nanochannels through the stratum corneum of the area of skin, (2) applying a composition comprising the agent to the prepared area of skin, and (3) causing an effective amount of the agent to deliver to the patient through the nanopores or nanochannels in the stratum corneum.

In some embodiments, the method of delivering an agent described herein includes: (1) applying a composition comprising the agent to an area of skin, (2) applying an application mechanism to a microdevice to cause the microdevice to contact the area of skin to generate a plurality of nanopores or nanochannels through the stratum corneum of the area of skin, and (3) causing an effective amount of the agent to deliver to the patient through the nanopores or nanochannels in the stratum corneum.

In some embodiments, the method of delivering an agent described herein includes: (1) applying an application mechanism to a microdevice to cause the microdevice to contact an area of skin to generate a prepared area of skin comprising a plurality of nanopores or nanochannels through the stratum corneum of the area of skin, and (2) causing an effective amount of an agent to deliver to the patient through the nanopores or nanochannels in the stratum corneum. The microstructure is coated with a composition comprising the agent.

### Skin Structure

Skin has a biological barrier called stratum corneum in its outer layer. This layer of about 20 microns thick prevents most of the molecules from penetrating through the skin. The layer below the stratum corneum is called viable epidermis. Epidermis is between 50 to 100 micron thick. The viable epidermis layer has no blood vessels and the molecules in this layer can be transported to and from the dermis, a layer under the viable epidermis, which is between 1 to 3 mm thick. There are blood vessels, lymphatics and nerves in dermis layer. To date, for example, a skin patch is only able to deliver drug molecules of less than 500 Da. In addition, these small molecules are typically limited to hydrophobic ones.

### Requirement of Delivery of Drugs, Vaccines and Cosmetic Substances

Successful transdermal delivery of therapeutic drugs, vaccines and cosmetic substances needs a way to transport molecules, especially large molecules through the skin barrier, stratum corneum. The substance can be delivered into the skin in any form acceptable to pharmaceutical requirements, but a gel composition is preferred to achieve controlled release of active ingredients.

The microdevice described herein can be used for effective transdermal delivery of an agent or a combination of agents. The microdevice can be a microdevice array comprises a plurality of microstructures formed of a metallic, semi-conductor, glass, ceramic, or polymeric material. In some embodiments, the microdevice can be microneedle, microknife, or microblade. In some embodiments, the microdevice comprising microstructures having a nanoscale tip or edge and a microscale body.

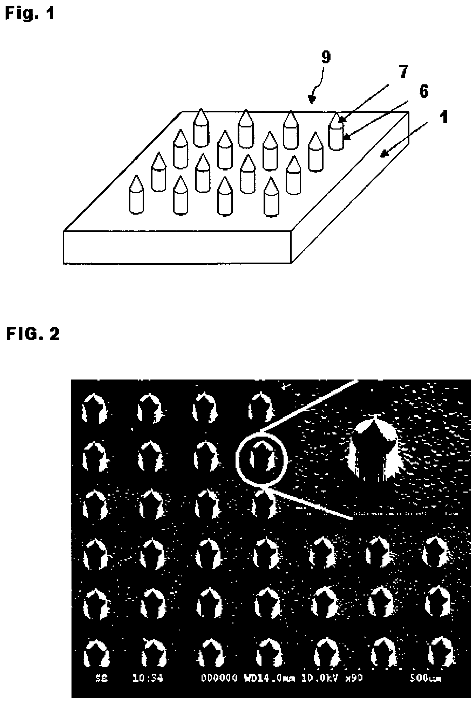

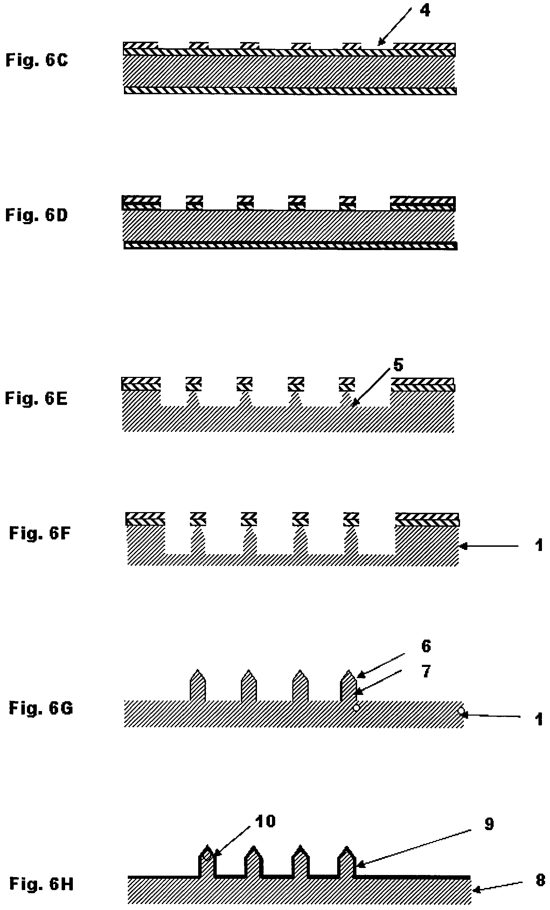

Referring to Figure 1, the preferred embodiment is a microdevice 9, having a body 7 and a sharp tip 6 in the case of microneedles or sharp edge 6 in the case of microblades and microknives. The body 7 and sharp tip 6, being out-of-plane on the substrate 1 are covered by a biocompatible coating 8 as illustrated in Figure 6H. They may have embedded nanochannels, connecting to microreservoirs where the active substances are stored.

Aspect-ratio is defined as the ratio of the depth or height of a structure to its lateral dimension. High-aspect-ratio microstructures (HARMS) typically have an aspect ratio higher than about 5:1 and they may be useful for a variety of purposes. In the current invention, the tip of microneedle 6 or the edge of the microblade and microknife 6 needs to be sharp in order to lower the insertion force, while the body of microdevice 7 should be high enough to allow it to completely penetrate stratum corneum. A typical size of the needle tip or width of edge on microblades and microknives is smaller than 10 microns, preferably smaller than 5 microns and the height of the microdevices is higher than 20 microns, preferably higher than 50 microns. The aspect ratio of these microdevices, in a preferred embodiment of the current invention, are higher than 10:1 with the size of the tip and edge smaller than 5 microns and the height of microdevices higher than 50 microns. HARMS can thus be used to fabricate microdevices including microneedles, microblades, and microknives for drug delivery through skin or body fluids extraction out of skin. Another example of HARMS is nanochannels for microfluidic manipulation and transport. HARMS is typically made by Micro-ElectroMechanical Systems (MEMS) and nanofabrication technology that involves a number of thin film deposition, photolithography, etching and electroplating, injection molding, hot embossing, self-assembly, as well as LIGA process.

### The Microneedles

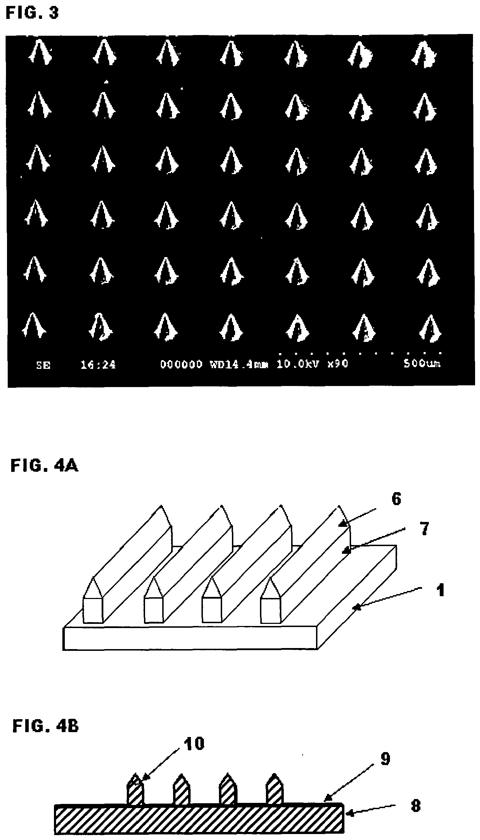

The microneedle devices disclosed herein can contain one or more microneedles. The length of the microneedle is typically in the range of 20-500 microns, sufficient to pierce through the outer skin barrier layer and deliver molecules to viable epidermis or even deeper. The diameter of a single microneedle is typically in the range of 30-300 microns with a sharp tip of less than 10 microns to cause little comfort to the patients while maintaining mechanical integrity. In one embodiment of the invention, the needle tip 6 is less than 2 microns and the height of the needle shaft 7 is about 100 microns. The aspect ratio is 50:1. Referring to Figure 6H, the angle of the tip 10 is between 30 to 75 degree, typically between 45-72 degree. Figure 2 shows a micrograph of microneedle arrays fabricated by this method with a zoom in view of a single microneedle that has a base diameter of about 80 microns. In another embodiment, Figure 3 provides a micrograph of a "pyramid-like" microneedles with a base size of about 80 microns. The size of the microneedles is bigger for collection of body fluids and they need to reach the dermis layer in the skin. In one embodiment of the current invention, the inner diameter of needle tip is about 10 microns and the height of needle is about 1200 microns to allow sufficient extraction of body fluids. The aspect ratio is 120:1.

### The Microblades and Microknives

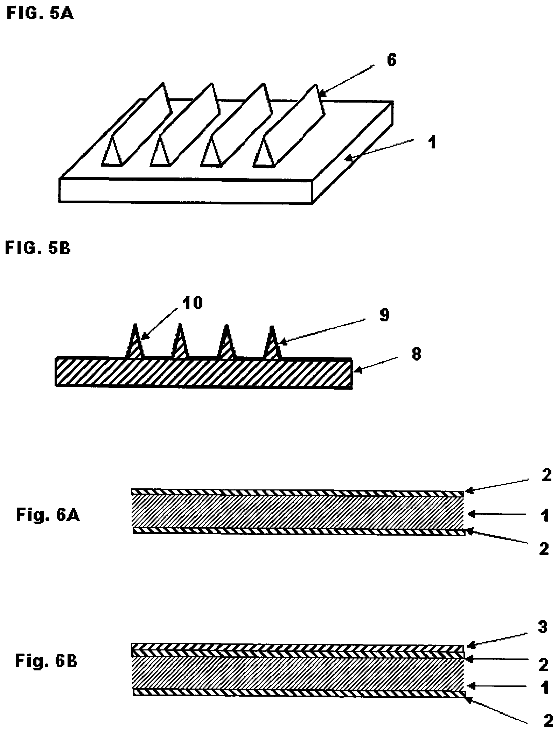

As shown in Figures 4 and 5, the microblades and microknives disclosed herein can contain one or more blades or knives. The sharp edge 6 of these devices is below 10 microns wide and the height of the body is more than 100 microns. In a preferred embodiment of the current invention, the edge 6 is below 3 microns and the body height 7 is about 150 microns. The skin contact area is about 0.003 mm x 1 mm for each microblade or microknife. The leading angle 10 of the blade edge is between 10 to 75 degree, preferably between 20-72 degree. It will become apparent from the following detailed description of the examples that the difference in manufacturing of microblade and microknife with respect to microneedle is only the shape of the mask using a cross-sectional illustration of the fabrication process disclosed in Figure 6A-6H.

### Materials and Device Sterilization

The devices can be made of many different materials or their combinations, including metals, ceramics, polymers and glass. Examples of the materials are titanium, stainless steel, nickel, alloy of nickel-iron, silicon, silicon oxide, glass, polymethyl methacrylate (PMMA), polyaryletherketone, nylon, PET, poly(lactic acid), poly(glycolic acid) (PGA), poly(lactic-co-glycolic acid) (PLGA), polycarbonate, and polystyrene. It should have enough mechanical strength to penetrate skin without break and buckle while ensure delivery of drugs, or collect of biological fluids. They can be sterilizable using established protocols (see, for example, moist heat, ethylene oxide or radiation sterilization as stated by ANSI/AAMI/ISO 11134:1993, ANSI/AAMI/ISO 11135:1994 and ANSI/AAMI/ISO 11137:1994).

### The Microchannels

High-aspect-ratio microchannels can be embedded in microdevices to allow flexible manipulation of microfluidics and connect microneedles to other functional blocks such as drug reservoirs. Microchannels can be made of many different materials or their combinations, including metals, ceramics, polymers and glass.

### The Microreservoirs

The microdevices can be connected to a reservoir with drug molecules and their proper compositions in liquid or solid forms. Microreservoir can be made of many different materials or their combinations, including metals, ceramics, polymers and glass. In a preferred embodiment, the microneedles are solid and the microreservoir is connected to the skin surface through microchannels. The gel containing active component can be dissolved by moisture evaporated from skin and the active substance can diffusion into skin through conduits opened along the interface of skin and microneedles. In another embodiment, the microneedles are hollow and they can be connected with drug reservoir through various microchannels. The reservoir can be made of natural polymers, deformable elastic polymers, metals and ceramics as listed above.

### Method of Use

The device described herein can be used for transdermal delivery of an agent or a combination of agents to treat, prevent, or ameliorate a body condition in need of treatment. The method generally includes treating a skin site of delivery with a microdevice described herein, and delivery an agent to the body of a mammal (e.g., a user or patient).

In some embodiments, the agent can be included in the microdevice as a coating with or without a carrier. In these embodiments, the agent can be delivered with the microdevice being attached to the site of delivery until a desired quantity or duration of delivery is achieved.

In some embodiments, the agent can be separate from the microdevice. In these embodiments, the skin site chosen for delivery the agent can pre-treated with the microdevice. The agent can then be applied to the skin site of delivery to allow the agent to penetrate into the body of a user or patient.

The body condition can be a medical condition or a cosmetic condition. Representative medical conditions include, but are not limited to, AIDS, breast cancer, melanoma, liver cancer, lung cancer, blood cancer, pituitary tumors, other cancers, flu, infection, blood disease, cardiac disease, back pain, neck pain, body pain, general pain, arthritis, osteoporosis, headache, depression, smoke, alcoholic, overweight and obesity, menopause, facial hair growth, balding, polycystic ovary syndrome, need of inoculation, need of anesthetics and in particular dermal disease. Representative cosmetic conditions include, but are not limited to, skin aging, skin wrinkle, dark spot, skin discoloration, moisturizing, skin lightening, skin whitening, skin firming, skin lifting, acne, wart, infection, irritation, dry skin and oily skin.

The microdevices of this invention are designed as disposable or re-usable devices. In one embodiment, the microdevices are disposable. Depending on whether the microdevices have coating of active substances on them or not, there are three categories of applications in the delivery of drugs, cosmetic substances and vaccines in the preferred embodiment.

For delivery of a drug, vaccine or cosmetic substance, in one embodiment, the microdevices can be used to perforate or scratch stratum corneum. They are then removed immediately and a formulation of an active substance such as a skin patch, cream, ointment, or lotion with the active substance is applied to the microdevice treated area right away. The formulation will stay on the skin for a pre-defined period, providing sustainable controlled release of an agent such as a drug, or a combination of agents.

Another embodiment is to store the active agents, as defined below, in the substrate and rely on passive diffusion when the microdevice is in touch with skin.

In yet a further embodiment, one can pre-coat microneedle shaft with a composition that contains active substances. The coated microneedles are applied to the skin and stay on the skin for the entire period of treatment. The rate of through skin transport can be measured using in vitro or in vivo methods known in the art.

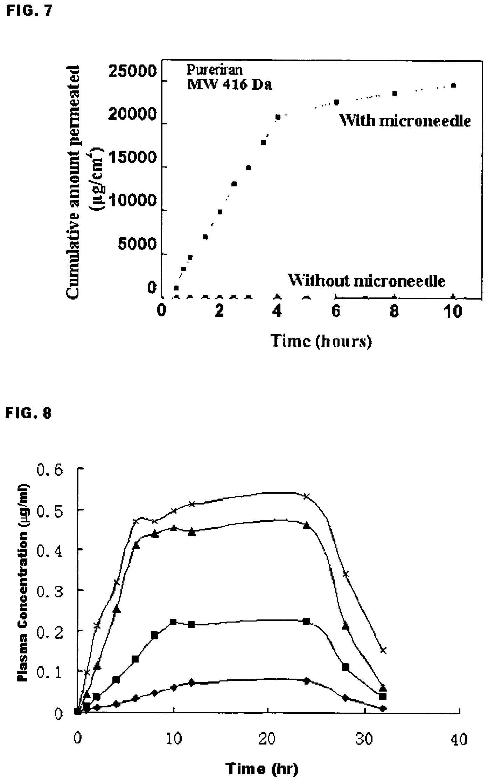

Referring to Figures 7-10, the microdevices disclosed herein are effective in increasing the through skin diffusion of molecules, especially therapeutic molecules with molecular weight higher than 500 Daltons and hydrophilic molecules to transport through skin barrier. As it will be further explained in the Examples, the enhancement of transdermal transport was also observed for small molecules with molecular weight lower than 500 Daltons as shown in Figure 7, as well as large molecules with molecular weight higher than 500. Because of the height of microneedles and microblades is limited, it will not reach the nerve-rich dermis layer and cause any discomfort to the subject.

### Active Agent

Active agents or active substances that can be delivered using microdevices are therapeutic agents. The term "therapeutic agent" is used here to refer to active agent that can treat, prevent, and ameliorate a body condition or skin condition that needs treatment. A list of examples includes: drugs, vaccines, peptides, proteins, genes, DNAs, nutraceuticals and cosmetics. The drugs can be administered topically and at whole system level. Examples of the drugs as active agents include, but not limited to antibiotics, hormones, steroids, anti-inflammatory drugs, protein drugs, DNA drugs whether natural or synthesized, such as Recombinant Erythropoietin (rhEPO), Taxol ®, Interferon-alpha-lb, Interferon beta, Interferon gamma, Emla®, Fluorouracil, Lidocaine, Salicylic acid, Pureriran, eflornithine hydrochloride, spironolactone, flutamide, insulin, nanoparticle drugs, Epidural, recombinant human parathyroid hormone, growth hormone, thyroid, cortisol, estrogen, progesterone, and testosterone. Examples of vaccines active agents include, but not limited to: vaccine against influenza (flu), diphtheria, tetanus, pertussis (DTaP), measles, mumps, rubella (MMR), hepatitis B, polio, haemophilus influenzae type b, chickenpox, tuberculosis, anthrax, yellow fever, rabies, AIDS, cancers, meningococcus, SARS and cholera. More examples of cosmetic substances as active agents include, but not limited to: botulinum toxin type A, hyaluronic acid and its derivatives, acetyl hexapeptide-3, vitamin A, vitamin C, vitamin E, alpha-hydroxyacids, collagen and hormones. Diagnostic reagents are also included. Examples include, but not limited to, quantum dots, functionalized nanoparticles, magnetic particles for diagnostic purpose.

The dosage of the agent can vary according to the medical conditions. The effective amount of an agent has been well established in the art can be publicly available. Such information can be obtained from the U.S. Food and Drug Administration (FDA), e.g., FDA website. For example, LidoDerm® info can be found in this link: http://www.fda.gov/medwaTCH/SAFETY/2006/Apr PIs/Lidoderm PI.pdf#search=%22li doderm%20dosage%22.

In some embodiments, the agent is a pain relieving drug for neuropathic or nociceptive pain management. Such pain relieving drug includes, but is not limited to, Lidocaine; Prilocaine, Tetracaine, Ibuprofen; Acetaminophen; Capsaicin; EMLA®; Tramadol (Ultram); Gabapentin, Tramadol hydrochloride, Corticosteroids, Sufentanil, Clonidine, Bupivacaine, Tricyclic antidepressants, opioid analgesics such as morphine, Hydromorphone, naloxone (Narcan), Talwin, Nubain, Stadol, Fentanyl, Meperidine, Hydrocodone, Codeine, Oxycodone; non-selective NSAIDs such as Celecoxib (Celebrex), rofecoxib (Vioxx), valdecoxib (Bextra); or combinations thereof. In some embodiments, the pain relieving drug described herein can specifically exclude any of the drug/agents listed herein.

In some embodiment, the active agent can be muscle relaxants, which include, but are but not limited to, Benzodiazepines; Methocarbamol; Carisoprodol; Chlorzoxazone; Metaxalone; Cyclobenzaprine, or combinations thereof. In some embodiments, the muscle relaxants described herein can specifically exclude any of the drug/agents listed herein.

### Drug delivery

In one aspect, the present invention provides a device 10 for delivery of therapeutic active agent as defined above across the skin barrier, stratum corneum layer. Once the substances pass the stratum corneum, there is less resistance for the substances to diffuse into the subsequent layers of the skin: epidermis and dermis. The substances will be absorbed by micro blood vessels and lymphatics in the dermis layer and delivered to entire human body. Microdevices disclosed in the current invention can enhance through skin penetration of molecules of molecular weight lower than 500 Dalton as shown in Figure 7. Microdevices can also enable through skin transport of large molecules of molecular weight higher than 500 Dalton as shown in Figures 8, 9 and 10. The molecular weight of Bovine Serum Albumin is 66,000 Dalton. The molecular weight of Botulinum Toxin Type A is 150,000 Dalton and the molecular weight of Interferon-Alpha-lb is 17,000 Dalton.

In some embodiments, the drug delivery of the present invention can be achieved by preparing an area of skin to generate a prepared area of skin and then applying an agent or drug to the prepared area of skin to allow a pre-defined amount of the drug or agent to pass through the stratum corneum of the parepared area of skin.

In some embodiments, the prepared area of skin can be prepared using a device, e.g., a spring-powered mechanical applicator to apply microneedles to an area of skin. The mechanical applicator can be any structure or design and can cause a mechanical force to be applied to the microneedle against the area of skin to generate pores or channels in the stratum comeum in the area of skin in a pre-defined size and depth. The size and depth of the pores or channels can provide for controlling the amount of an agent or drug of delivery.

In some embodiments, the prepared area of skin can be further treated using an ultrasound device or a mechanical vibrator to apply microneedles to an area of skin. The ultrasound device or mechanical vibrator can cause a pre-set mechanical force to be applied to the microneedle against the area of skin to generate pores or channels in the stratum corneum in the area of skin in a pre-determined size and depth. The size and depth of the pores or channels can provide for controlling the amount of an agent or drug of delivery. It is noteworthy that the ultrasound device or mechanical vibrator can be an effective way to perforate an elastic skin tissue to generate pores or channels in a pre-defined size and/or depth.

In some embodiments, the prepared area of skin can be prepared in a pre-defined size or dimension (e.g., a dimension of 1 cm x 1 cm) using an array of microknives or microblades by slicing or lacerating the stratum corneum in an area of skin to generate nanochannels in a pre-defined depth and/or dimension. The dimension and/or depth of the laceration and the dimension of the prepared area of skin can provide for controlling the amount of an agent or drug.

Allowing an agent or drug to pass through the stratum corneum of a prepared area of skin can be achieved by a variety of mechanisms. For example, the allowing can be achieved by diffusion of the agent or drug from a topical composition (e.g., a formulation such as a skin patch, cream, ointment, or lotion) into the body of a patient or user via the prepared area of skin. In some embodiments, the allowing can be achieved by a driving mechanism, for example, iontophoresis, sonophoresis, radiofrequency (RF) or heat or a combination of these to actively drive agents through the skin.

Iontophoresis, sonophoresis, radiofrequency (RF) or heat are well developed mechanisms for promoting or enhancing drug delivery. Some examples of iontophoresis systems in drug delivery are described in http://www.vyteris.com and http://www.iomed.com. Some examples of sonophoresis systems in drug delivery are described in www.sontra.com (Becker B, Helfrich S, Baker E, et al. Ultrasound with topical anesthetic rapidly decreases pain of intravenous cannulation. Academic Emergency Medicine 2005; 12:289-295; Katz N, Shapiro D, Herrmann T, et al., Rapid onset of cutaneous anesthesia with EMLA cream after pretreaatment with a new ultrasound-emitting device. Pain Trials Center, Brigham and Women's Hospital, Boston, Massachusetts; Mitragotri S, Kost J, Low frequency sonophoresis: A Review. Advanced Drug Delivery Reviews 2004; 56:589-601. Some examples of RF systems in drug delivery are described in http://www.transpharma-medical.com/references.html. Some examples of drug delivery systems using heat are described in http://www.zars.com.

### Topical delivery of cosmetic substances

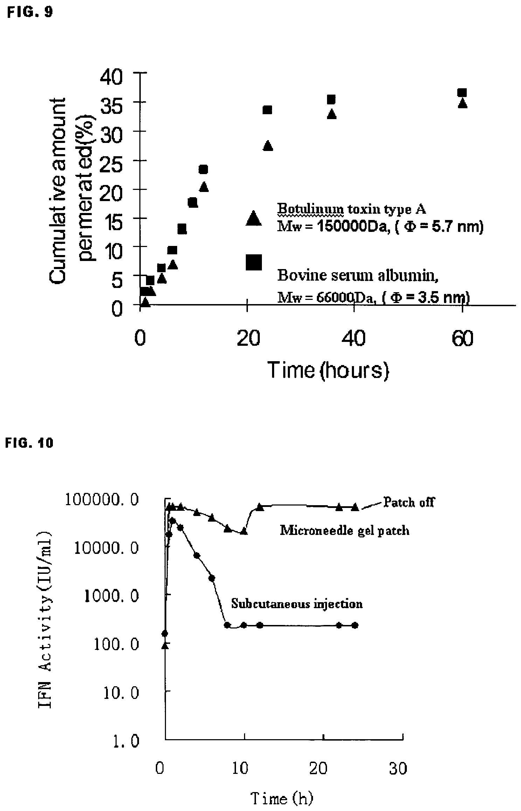

It is known to one in the art that certain substances have specific functions as cosmetics. For example, Botulinum Toxin Type A is a toxin that blocks neuromuscular transmission when it is injected in small amounts (e.g., 10 units per 0.1 ml injection volume) into specific muscles to treat and reduce wrinkles on the face. The maximum dosage recommended as a single injection for any one muscle at any spot is 25 units. If overdosed or the injection is incorrectly performed, the patient can be left with an immobile face or droopy eyelids till the effect of the injection wears off. The side effects include numbness, swelling and headaches. Administered through microdevices disclosed in the current invention, it is possible to provide a controlled release of Botulinum Toxin Type A and keep an optimal local concentration to achieve the best result while minimize the side effects. In a preferred embodiment of this invention, gel patch with botulinum toxin type A is applied to the skin pre-treated with microneedle array. A significant increase in through skin penetration of botulinum toxin type A was observed as detailed in Figure 9. No through skin transport was observed without application of microdevices. More examples were provided in the above "active agents" section.

It is also evident that through skin transport on microdevices treated skin has less dependency on molecular weight as observed in Figure 9. Using the methods described herein, practically, any cosmetic substances can be delivered using microdevices herein. Local concentration can be adjusted through loading and composition for controlled release. In one embodiment of this invention, one can deliver hyaluronic acid gel through diffusion enhanced by microdevices. Hyaluronic acid is a substance that exists naturally in the body. A major important function of hyaluronic acid is to carry and bind water molecules. Stabilized non-animal hyaluronic acid does not contain animal protein and does not require a skin test prior to treatment. It is thus a preferred embodiment of this invention to use microdevices to delivery locally stabilized non-animal hyaluronic acid to treat wrinkles and facial lines.

Yet, in a further embodiment of this invention, one can locally delivery collagen by microneedles, e.g., for allergic skin test and controlled release of collagen into the skin.

Yet, another embodiment of this invention is to provide for local delivery of acetyl hexapeptide-3. This molecule is a non-toxic, non-irritant compound that modulates the excessive stimulation of the facial muscles, relaxing facial tension and it can reduce and prevent the formation of new wrinkles due to over-stimulation of facial muscles. More examples include but not limited to: vitamin A, vitamin C, vitamin E, alpha-hydroxyacids, hormones, or combinations thereof.

### Delivery of vaccines

In some embodiments, the microdevice provided herein can be used for topical delivery of vaccines below the stratum corneum layer. The type of vaccines includes conventional vaccines as well as protein, peptide, DNA vaccines and the like as previously described. Vaccination can be performed by treating a skin site with the microdevice and then delivering a vaccine composition to a user.

### Pain management

In some embodiments, the microdevice described herein can be used for pain management. The microdevice can be used to facilitate transdermal delivery of a pain relieving agent or a combination of them so as to treat, reduce or prevent pain. In some embodiments, a skin site can be treated with the microdevice and then a pain relieving agent or drug composition can be applied to the treated site, allowing transdermal delivery of these agents to a user.

The pain relieving agent can be any pain relieving agent approved by FDA or used in medical practice elsewhere in the world. In some embodiments, the pain relieving drug can be, but are not limited to, NSAIDs, COX-2 inhibitors, steroids, muscle relaxants. Specifically, such as Lidocaine; Prilocaine, Tetracaine, Ibuprofen; Acetaminophen; Capsaicin; EMLA®; Tramadol (Ultram); Gabapentin, Tramadol hydrochloride, Corticosteroids, Sufentanil, Clonidine, Bupivacaine, Tricyclic antidepressants, opioid analgesics such as morphine, Hydromorphone, naloxone (Narcan), Talwin, Nubain, Stadol, Fentanyl, Meperidine, Hydrocodone, Codeine, Oxycodone; non-selective NSAIDs such as Celecoxib (Celebrex), rofecoxib (Vioxx), valdecoxib (Bextra); or combinations thereof. In some embodiments, the pain relieving drug described herein can specifically exclude any of the drug/agents listed herein.

The pain management can be carried out according to a management regime prescribed by a treating doctor. For example, in some embodiment, the pain management is chronic or acute pain management. The pain management regime can be but not limited to, lower back pain, post-herpetic neuralgia, cancer pain, diabetic neuropathy, phantom limb pain, spinal stenosis/sciatica, spinal mets, HIV pain, pain caused invasive medical procedures such as needle injection, cannulation.

### Controlled Release

The microdevices need to deliver drug molecules through skin at a rate that is sufficient to maintain a therapeutic useful concentration in plasma. The size, density of the microdevices can be adjusted to meet the delivery requirement. The microdevices can be further coated with a composition that contains active therapeutic molecules, or vaccines, or cosmetic substances, together with polymer binders such as chitosan, carbopol 934P, cellulose and starch to form a dry film. Additional additives of rheology modifiers, surface active agents, stabilizer, rehydration agents may be used. The special composition can control the dissolve rate of the active drug molecule and regulate the drug release rate. One example for controlled release of bovine serum albumin is shown in Figure 8. The microdevices may be integrated with embedded microfluidic channels that connect to microreservoirs.

### The Integrated Sensors

It is another aspect of the invention to provide a device in which clinical biosensor and/or sensor arrays are fabricated in the close vicinity of these HARMS structures. For example, microneedle can collect an extremely low sample volume of body fluids from a patient and allow rapid point-of-care analysis of body fluids. In one embodiment, the sample volume extracted is below 0.1 microliter, typically around 0.01 microliter.

### Methods for HARMS Fabrication

The HARMS were fabricated using MEMS microfabrication technology. The typical fabrication process involved lithography, wet etch and dry etch, thin film deposition and growth, electroplating, as well as injection molding and hot embossing. One example of fabrication method was to use Bosch process that allowed deep Si etch (www.oxfordplasma.de/process/sibo_1.htm). It formed HARMS suitable either as device body or mold for further processing. The aspect ratio was higher than 5:1, independent to feature size and pattern shape as long as the features can be defined by lithography. Another fabrication method was KOH or TMAH wet etch of single crystal Si substrate that is <100> orientation or <110> orientation. Yet another fabrication method was using HF solution to electrochemically form porous Si structures (www.techfak.uni-kiel.de/matwis/amat/poren/ps.html). Metals was used for the fabrication of HARMS through a maskless process called electropolishing starting from a structure fabricated by traditional machining methods such as cutting, electro-discharge machining, milling, grinding, polishing and drilling (www.najet.com and www.fischion.com/product_support/model_110_application notes.asp). Use of any single method herein or a combination of these methods as further disclosed in the examples below led to the form of desired HARMS disclosed in the current invention.

### EXAMPLES

### Example 1. Fabrication of Si microneedles

Bosch etch process is a widely used MEMS process that is known in the art to etch deep vias and trenches. The current invention disclosed a method that involved isotropic etch first to form microneedle tip 6 and second anisotropic Bosch etch later to form microneedle shaft 7 as shown in Figure 6G.

Referring to Figure 6A, a silicon wafer 1 of any crystal orientation with about 1 micron of thermal oxide 2 coated on both sides was used as substrate. Doping level in the silicon substrate was found to be not critical. A chromium layer with thickness ranging between 0.2 to 20 nm was sputtered onto the substrate. A layer of photoreist 3 was applied to front side of the wafer as shown in Figure 6B. It was patterned into a number of circular islands with a diameter that was equal to the base of the microneedles as shown in Figure 6C. The shape of the pattern was changed to rectangular to form microknives and microblades. The Cr etch was done using common method that have been known in the art. Cr film served as hard mask to etch part of oxide and silicon substrate 4. Oxide 2 was patterned, as shown in Figure 6D by either buffered oxide etch in solution or dry etch using CHF₃ or CF₄ as etching gases. In a preferred embodiment of the current invention, an isotropic etch of Si was done using SF₆ only while the Cr and oxide mask materials were still there as shown in Figure 6E. The undercut of Si substrate 1 formed the tip 5 of the microneedles as shown in Figure 6E. Once Si undercut reached the stage with about 20 microns left, Bosch process was used to perform alternative deposition and etch cycles until the etch depth reached the pre-defined length of microneedles as shown in Figure 6F. The final shape of microneedles was formed by a short wet etch using a mixture of acetic acid, nitric acid and fluoric acid, known as HNA to the people in the art. HNA dip helped release the hard mask and sharpen the tip 6 of the microneedle as shown in Figure 6G. The formed microneedle was coated with about 200-500 A Ti coating to improve biocompatibility as shown in Figure 6H. A micrograph of microneedles fabricated in this way is shown in Figure 2.

### Example 2. Fabrication of Si microneedles

The current invention also disclosed a method that did anisotropic etch first to form microneedle body and switch to orientation dependent etch (ODE) later to form microneedle shaft and tip.

A silicon wafer of <100> orientation with 0.5-3 micron of thermal oxide coated on both side was used as substrate as shown in Figure 6A. The oxide was patterned into a number of circular or square islands with a diameter that was roughly equal to the base of the microneedles. In one example, the diameter of the circular pattern was about 100 micron. The oxide etch was done by either buffered oxide etch in solution or dry etch using CHF₃ or CF₄ as etching gases. Once silicon oxide etch was completed, Bosch process was used to perform alternative deposition and etch cycles until the etch depth reaches the pre-defined value of 30-500 µm, yielding a number of circular or square posts on the silicon substrate. The final shape of microneedles, as shown in Figure 6G was formed by KOH etch in a heated bath at a temperature between 20-90 degree. The KOH concentration was between 10% to 80%. Typically, the etch temperature was between 50 to 80 degree and potassium hydroxide (KOH) concentration was between 20-50%. Tetramethyl ammonium hydroxide (TMAH) or sodium hydroxide was used to replace KOH to give similar results. Typical etch condition was 20%-40 % TMAH at about 80 degree. About 500 A Ti coating was applied to the formed microneedles to improve biocompatibility as shown in Figure 6H. Figure 3 shows microneedle array fabricated in this way.

### Example 3. Fabrication of Microblades and Microknives

As shown in Figure 6C, if the pattern is a rectangular shape, the microdevices formed according to the process flow disclosed in the Examples 1 and 2 become microknives and microblades.

### Example 4. Fabrication of Polymer Microneedles

A stainless steel piece was used as cathode. A patterned conductive piece with round posts on it was used as anode. The height of the posts was comparable to microneedle height. Both electrodes were immersed in an electrolyte and a voltage of 0.5-5 V was applied to them, a selective area of cathode was dissolved away, leaving behind arrays of cavity. Once the etch was complete, the stainless steel piece with microneedle body in the shape of cavity was obtained. It was used as mold insert for micro-injection molding to obtain polymeric microneedles, using materials listed previously.

### Example 5. Fabrication of Metal Microneedles

A metal piece such as stainless steel, or Ti or Ni was used as cathode. A conductive piece with a grid pattern on it was used as anode. The size of each square was comparable to base of microneedle. When both electrodes were immersed in an electrolyte and a voltage of 0.5-5 V was applied to them, a selective area of cathode were dissolved away, leaving behind arrays of microneedles. The tips 6 of the microneedles were sharpened by electropolishing, yielding very sharp metal microneedle arrays.

### Example 6. Application of Microdevices

In one embodiment, Chitosan was purified before use as following: A 20 g of chitosan was dissolved in 1L of 1% acetic acid solution. After it was filtered under reduced pressure, 1 M NaOH was added into the filtrate to produce precipitate. The precipitate was washed repeatedly with de-ionized water until the pH value of rinse water was about 7. The precipitate was dried in a vacuum dry box under reduced pressure. The purified chitosan was milled to powder and was used in preparing stock solution that may further contain additives of rheology modifiers, surface active agents, stabilizer, rehydration agents and a combination of thereof to form a composition. The known volume of chitosan stock solution containing known amount of active agent was prepared by dissolving active agent in a chitosan stock solution. Skin patch containing active agent was prepared using the casting method in cleanroom environment. The formulation prepared this way was let dry at room temperature.

The application of microdevice enabled skin patch was a simple two-step procedure. The first step was the perforation or scratch of skin using a microneedles or microknives and microblades with a plastic handler attached to them on the backside with no high aspect ratio microstructures. These microdevices were coated with a biocompatible film and had no active agent on them. This step can also be replaced using a spring-powered mechanical device with microneedle or microknives or microblades loaded. For example, a lancing device with a disposable microneedle attached to the moving end. The second step was the application of skin patch prepared using a method disclosed herein to the microdevice treated skin area. Spring-powered devices provided results with better consistency.

In another embodiment, the microdevice arrays were coated with chitosan composition and let dry in a dust-free atmosphere at room temperature. The microdevice containing active agent was applied to the skin manually or using a spring powered device. Adhesive tape was used to keep the microdevice on the skin during the entire treatment cycle.

### Example 7. Application of microdevices in pain management

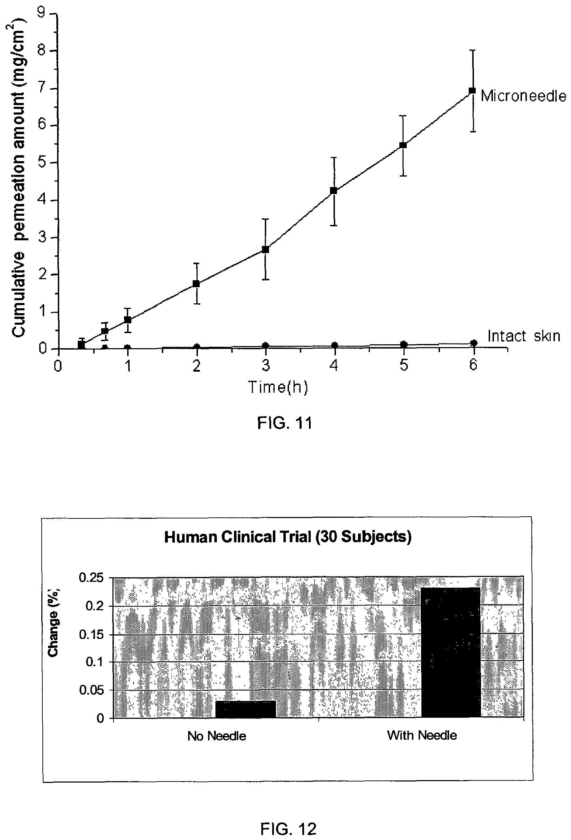

A microdevice described herein was tested for use in pain management. Figure 11 shows some *in vivo* test result showing that lidocaine, an anesthetic agent, can penetrate skin 10 times faster on a microneedle pre-treated skin. As seen from Figure 11, pain management using the device provided herein, as compared to prior art, has at least these two advantages: (1) rapid onset, and (2) about ten times improvement in transdermal drug delivery.

### Example 8. Application of microdevices in pain management

A microdevice described herein was tested for use in pain management. Figure 12 shows test result on 30 human subjects showing about 7 times improvement in the improvement of the pain score with microneedle assisted lidocaine patch, as compared to placebo group that has lidocaine patch only.

### Example 9. Method of transdermal application of microdevice array

The conventional method of applying a therapeutic agent by microdevice array is to coat microdevice array such as microneedle array with drug and then apply drug coated device to skin to deliver drugs transdermally. The microdevice of the present invention were used for transdermal delivery of a drug in three different combinations where the microdevice and drugs can be separate - in that the drug does not have to, although it can, be coated on the device or otherwise placed on or within the microdevice: (1) one can apply drug coated device; (2) one can apply microneedle first to prepare the skin, then apply lotion, cream or ointment or patch loaded with drugs to the prepared skin; and (3) one can also apply drugs to the skin areas first, then perforate stratum corneum using the fabricated devices. All three method can enhance trasdermal drug transport through skin.

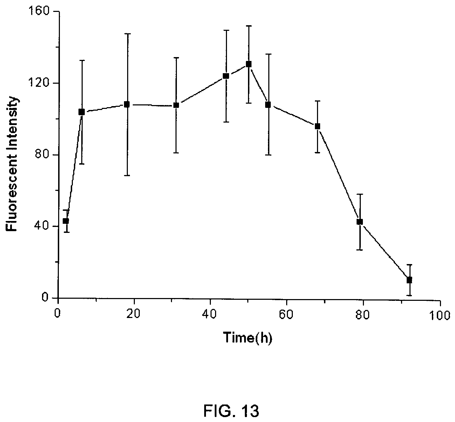

Figure 13 shows blood concentration of BSA indicated by fluorescence intensity, which was delivered to rabbit skin using a microneedle array of the present invention. The BSA was delivered by applying microneedle array and then applying fluorescence labeled BSA to the rabbit skin and monitor the blood concentration of BSA using fluorescence intensity. Figure 13 shows blood concentration of BSA as monitored by fluorescence as a function of time. BSA was collected after the treatment of our microneedle array, then apply a BSA skin patch, cream, ointment, or lotion to the rabbit skin. Figure 13 demonstrates that transdermally deliver FITC labeled (a fluorescence label) BSA (Mw = 66,000) for up to 60 hours on skin area pre-treated by microneedle array can be readily achieved.

While particular embodiments of the present invention have been shown and described, it will be obvious to those skilled in the art that changes and modifications can be made without departing from this invention in its broader aspects. Therefore, the appended claims are to encompass within their scope all such changes and modifications as fall within the scope of this invention.

## Claims

1. A system for delivering
an agent or a combination of agents, comprising:
a microdevice comprising an array of microstructures, wherein the microdevice has an aspect ratio of about 10:1 or higher,
an application mechanism for applying the microdevice to an area of skin to generate a prepared area of skin comprising a plurality of nanopores or nanochannels in stratum corneum of the prepared area of skin, and
a delivery mechanism for causing the agent or combination of agents to be delivered through the nanopores or nanochannels in the stratum corneum of the prepared area of skin,
wherein the application mechanism and/or delivery mechanism comprises a mechanical applicator, an ultrasound applicator, a mechanical vibrator, an iontophoresis device, a sonophoresis device, a radiofrequency (RF) device, or a heat generating device.

2. The system of claim 1, wherein the delivery mechanism comprises an iontophoresis device, a sonophoresis device, a radiofrequency (RF) device or a heat generating device.

3. The system of claim 1, wherein the microstructure comprises a microneedle, microblade, microknife, or a combination thereof.

4. The system of claim 1, wherein the microdevice comprises at least one skin-contacting area that is connected through microchannels to a reservoir containing the agent.

5. The system of claim 1, wherein the microstructure comprises microneedles, microknives or microblades coated with a thin layer of a composition comprising the agent.

6. The system of claim 1, wherein the agent is a natural or synthetic vaccine selected from the group consisting of vaccines, protein vaccines, peptide vaccines, gene vaccines and DNA vaccines, and
wherein the vaccine is against influenza (flu), diphtheria, tetanus, pertussis (DTaP), measles, mumps, rubella (MMR), hepatitis B, polio, haemophilus influenzae type b, chickenpox, tuberculosis, anthrax, yellow fever, rabies, AIDS, cancers, meningococcus, SARS or cholera.

7. The system of claim 1, wherein the agent is a pain relieving agent.

8. The system of claim 1, wherein the medical condition is chronic back pain.

9. The system of claim 1, wherein the medical condition is a cancer.

10. The system of claim 7, wherein the pain relieving agent is lidocaine, and wherein the delivery mechanism comprises a skin patch, cream, ointment, or lotion.

## Patentansprüche

1. Ein System zum Verabreichen eines Wirkstoffs oder einer Kombination von Wirkstoffen, umfassend:
eine Mikro-Vorrichtung, die eine Anordnung von Mikrostrukturen umfasst, wobei die Mikrostrukturen ein Aspektverhältnis von etwa 10:1 oder höher haben,
einen Anwendungs-Mechanismus zum Anwenden der Mikro-Vorrichtung auf einen Hautbereich zum Erzeugen eines präparierten Hautbereichs, der eine Mehrzahl von Nanoporen oder Nanokanälen im Stratum Corneum des präparierten Hautbereichs umfasst, und
einen Verabreichungsmechanismus , um die Verabreichung des Wirkstoffs oder der Kombination von Wirkstoffen durch die Nanoporen oder Nanokanäle des präparierten Hautbereichs zu bewirken,
wobei der Anwendungs-Mechanismus und/oder der Verabreichungsmechanismus einen mechanischen Applikator, einen Ultraschall-Applikator, einen mechanischen Vibrator, eine Iontophorese-Vorrichtung, eine Sonophorese-Vorrichtung, eine Hochfrequenz(HF)-Vorrichtung oder eine Wärme erzeugende Vorrichtung aufweist.

2. System nach Anspruch 1, wobei der Verabreichungsmechanismus eine Iontophorese-Vorrichtung, eine Sonophorese-Vorrichtung, eine Hochfrequenz(HF)-Vorrichtung oder eine Wärme erzeugende Vorrichtung aufweist.

3. System nach Anspruch 1, wobei die Mikrostruktur eine Mikronadel, eine Mikroschneide, ein Mikromesser oder eine Kombination davon umfasst.

4. System nach Anspruch 1, wobei die Mikro-Vorrichtung mindestens einen Hautkonktaktbereich aufweist, der durch Mikrokanäle mit einem Reservoir, das den Wirkstoff enthält, verbunden ist.

5. System nach Anspruch 1, wobei die Mikrostruktur Mikronadeln, Mikromesser oder Mikroschneiden aufweist, die mit einer dünnen Schicht einer Zusammensetzung, die den Wirkstoff enthält, bedeckt sind.

6. System nach Anspruch 1, wobei der Wirkstoff ein natürlicher oder synthetischer Impfstoff ist, ausgewählt aus der aus Impfstoffen, Protein-Impfstoffen, Peptid-Impfstoffen, Gen-Impfstoffen und DNA-Impfstoffen bestehenden Gruppe, und wobei der Impfstoff gegen Influenza (Grippe), Diphterie, Tetanus, Keuchhusten (DTaP), Masern, Mumps, Röteln (MMR), Hepatitis B, Polio, Haemophilus Influenzae Typ B, Windpocken, Tuberkulose, Anthrax, Gelbfieber, Tollwut, AIDS, Krebserkrankungen, Meningokokken, SARS oder Cholera ist.

7. System nach Anspruch 1, wobei der Wirkstoff ein schmerzlindernder Wirkstoff ist.

8. System nach Anspruch 1, wobei der medizinische Zustand chronische Rückenschmerzen sind.

9. System nach Anspruch 1, wobei der medizinische Zustand eine Krebserkrankung ist.

10. System nach Anspruch 7, wobei der schmerzlindernde Wirkstoff Lidocain ist, und wobei der Verabreichungsmechanismus ein Pflaster, eine Creme, eine Salbe oder eine Lotion umfasst.

## Revendications

1. Système d'administration
d'un agent ou d'une combinaison d'agents comprenant :
un micro-dispositif ayant un réseau de microstructures dans lequel,
le micro-dispositif a un rapport dimensionnel d'environ 10 : 1 ou plus,
un mécanisme d'application pour appliquer le micro-dispositif sur une région de peau, pour y générer une région de peau préparée comportant un ensemble de nanopores ou de nanocanaux dans la couche cornée de la zone préparée de la peau, et
un mécanisme de distribution pour distribuer l'agent ou la combinaison d'agents à travers les nanopores ou nanocanaux dans la couche cornée de la région de peau préparée,
dans lequel, le mécanisme d'application et/ou le mécanisme de distribution comportent un applicateur mécanique, un applicateur à ultrasons, un vibreur mécanique, un dispositif d'iontophorèse, un dispositif de sonophorèse, un dispositif de radiofréquence (RF) ou un dispositif chauffant.

2. Système selon la revendication 1,
dans lequel le mécanisme de distribution comporte un dispositif d'iontophorèse, un dispositif de sonophorèse, un dispositif de radiofréquences (RF) ou un dispositif chauffant.

3. Système selon la revendication 1,
dans lequel la microstructure comprend une micro aiguille, une micro lame, un micro-couteau ou une combinaison de ces moyens.

4. Système selon la revendication 1,
dans lequel le micro-dispositif comporte au moins une région de contact de peau qui est reliée par des micro-canaux à un réservoir contenant l'agent.

5. Système selon la revendication 1,
dans lequel la microstructure comporte des micro-aiguilles, des micro-couteaux ou des microlames revêtus d'une mince couche d'une composition comportant l'agent.

6. Système selon la revendication 1,
dans lequel l'agent est un vaccin naturel ou de synthèse choisi dans le groupe comprenant les vaccins, les vaccins de protéines, les vaccins de peptides, les vaccins de gênes et les vaccins DNA, et
dans lequel le vaccin est un vaccin contre la grippe, la diphtérie, le tétanos, le pertussis, (DTaP), la rougeole, les oreillons, la rubéole (MMR), l'hépatite B, la poliomyélite, l'hémophilie, la grippe de type B, la varicelle, la tuberculose, l'anthrax, la fièvre jaune, la rage, le SIDA, les cancers, le méningocoque, le SARS ou le choléra.

7. Système selon la revendication 1,
dans lequel l'agent est un agent antidouleur.

8. Système selon la revendication 1,
dans lequel la condition médicale est une douleur chronique au dos.

9. Système selon la revendication 1,
dans lequel la condition médicale est un cancer.

10. Système selon la revendication 7,
dans lequel l'agent antidouleur est de la lidocaïne et le mécanisme de distribution est une étiquette de peau, une crème, un onguent ou une lotion.
